# EUROPÄISCHE PATENTSCHRIFT

(11) **EP 3 082 751 B1**
(45) Veröffentlichungstag und Bekanntmachung des Hinweises auf die Patenterteilung: **19.09.2018**
(21) Anmeldenummer: 14790499.9
(22) Anmeldetag: 25.09.2014
(51) Int. Cl.: A61K 8/49, A61Q 5/10

(54) **TETRASUBSTITUIERTE DERIVATE DES PYRIMIDINS IN MITTELN ZUM OXIDATIVEN FÄRBEN VON KERATINFASERN**
TETRASUBSTITUTED PYRIMIDINE DERIVATIVES IN OXIDATIVE DYEING PREPARATIONS
PYRIMIDINES TÉTRASUBSTITUÉS& xA;DANS LES PRÉPARATIONS OXYDANTES DE TEINTURE

(30) Priorität: 19.12.2013 DE 102013226585
(43) Veröffentlichungstag der Anmeldung: 26.10.2016
(73) Patentinhaber: Henkel AG & Co. KGaA, 40589 Düsseldorf (DE)
(72) Erfinder: GEBERT-SCHWARZWÄLDER, Antje, 40545 Düsseldorf (DE); GIESA, Helmut, 40670 Meerbusch (DE); MOCH, Melanie, 41542 Dormagen (DE)
(86) Internationale Anmeldenummer: PCT/DE2014/200506
(87) Internationale Veröffentlichungsnummer: WO 2015/090292

(56) Entgegenhaltungen:
- EP-A1- 1 598 047
- DE-A1-102011 087 344
- US-A1- 2005 257 333
- US-A1- 2006 156 481

## Beschreibung

Die Erfindung betrifft ein Mittel zur oxidativen Färbung von keratinhaltigen Fasern, insbesondere menschlichen Haaren, welches als Oxidationsfarbstoffvorprodukt vom Entwicklertyp mindestens ein tetrasubstituiertes Derivat des Pyrimidins enthält. Ein weiterer Gegenstand der vorliegenden Erfindung ist eine Mehrkomponenten-Verpackungseinheit enthaltend das obige Mittel. Ein weiterer Gegenstand ist auch die Verwendung des Mittels zur Verbesserung des Egalisiervermögens, der Lichtechtheit und der Waschechtheit von oxidativen Färbungen

Die Veränderung von Form und Farbe der Haare stellt einen wichtigen Bereich der modernen Kosmetik dar. Zur modischen Farbgestaltung von Frisuren oder zur Kaschierung von ergrautem oder gar weißem Haar mit modischen oder natürlichen Farbtönen greift der Verbraucher zu farbverändernden Mitteln.

Zur Veränderung der Farbe von keratinischen Fasern, insbesondere menschlichen Haaren, kennt der Fachmann je nach Anforderungen an die Färbung bzw. Farbveränderung diverse Systeme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich generell durch intensive, hervorragende, lang anhaltende Färbeergebnisse aus. Für natürlich wirkende Färbungen kann eine Mischung aus einer größeren Zahl von Oxidationsfarbstoffvorprodukten eingesetzt werden, wobei in vielen Fällen weiterhin zusätzlich direktziehende Farbstoffe zur Nuancierung verwendet werden.

Oxidative Haarfärbemittel sind trotz ihrer vorteilhaften Färbeeigenschaften für den Anwender mit Nachteilen behaftet. Insbesondere wird für einige der gängigen Oxidationsfarbstoffvorprodukte, darunter p-Phenylendiamin, vermutet, für manche Verbraucher irritierend oder reizend zu wirken und dadurch Sensibilisierungen oder gar allergische Reaktionen auszulösen. Daher besteht für diese Substanzen noch weiterhin Verbesserungsbedarf hinsichtlich ihres physiologischen Verträglichkeitsprofils. Auf der Suche nach Ersatzstoffen wurden viele Verbindungen erforscht, die aber häufig unter anwendungstechnischen Problemen, insbesondere mangelndem Egalisiervermögen, leiden. Außerdem besteht trotz bereits hoch entwickelter Färbesysteme weiterhin Bedarf an Färbesystemen, die hervorragende Leuchtkraft und Intensität der Färbungen erreichen, und diese auch bei Einwirkung von äußeren Umwelteinflüssen wie Bestrahlung durch Tageslicht bzw. Haarwäschen, nicht verlieren.

Aufgabe der vorliegenden Erfindung ist es daher, die oben genannten Nachteile oxidativer Haarfärbemittel herabzusenken. Die Färbemittel sollen intensive Färbungen mit hoher Farbigkeit und mit einer guten Beständigkeit gegenüber äußeren Einflüssen, insbesondere mit guter Lichtechtheit und Waschechtheit, erzeugen, welche auch nach intensiver Bestrahlung mit Sonnenlicht und mehrmaligem Shampoonieren der Haare keine Farbabschwächung oder Farbverschiebung erleiden. Darüber hinaus sollen die Färbungen möglichst ein hervorragendes Egalisiervermögen aufweisen und wenig selektiv sein, d.h. auf unterschiedlich vorbehandeltem Haar möglichst gleichmäßige, einheitliche Färbeergebnisse erzielen. Außerdem sollen die Färbemittel ein toxikologisch vorteilhaftes Profil besitzen.

US 2005/257333 A1 beschreibt Mittel zum Färben von Haaren, die neben direktziehenden Farbstoffen auch ein heterozyklisches Oxidationsfarbstoffvorprodukt enthalten.

In DE 10 2011 087 344 A1 wird ein Vorbehandlungsmittel beschrieben, das im Verfahren vor einer oxidativen Färbung eingesetzt werden kann. Für die oxidative Färbung schlägt DE 10 2011 087 344 A1 generisch den Einsatz verschiedener Pyrimidinderivate vor.

EP 1 598 047 A1 betrifft Färbemittel mit direktziehenden Farbstoffen und Entwicklern vom Pyrazol-Typ. Zusätzlich können die Mittel auch weitere Entwickler vom Pyrimidin-Typ enthalten.

US 2006/156481 A1 beschreibt oxidative Färbemittel, die als Oxidationsfarbstoffvorprodukte die N-Oxide von Stickstoffheterozyklen, darunter auch die 1-Oxide von Pyrimidinen enthalten.

Es wurde nun gefunden, dass sich bestimmte tetrasubstituierte Pyrimidinderivate hervorragend als Oxidationsfarbstoffvorprodukte vom Entwicklertyp zum Färben von keratinhaltigen Fasern eignen. Mit den neuartigen Pyrimidinderivaten lassen sich insbesondere im Bereich modischer Rot- , Rotbraun- und Violettnuancen intensive Färbungen mit gutem Egalisiervermögen, guter Lichtechtheit und guter Waschechthteit erzeugen.

Ein erster Gegenstand der Erfindung ist ein Mittel zur oxidativen Färbung keratinischer Fasern, enthaltend in einem kosmetischen Träger als Oxidationsfarbstoffvorprodukt vom Entwickler-Typ mindestens eine Verbindung der Formel (I), 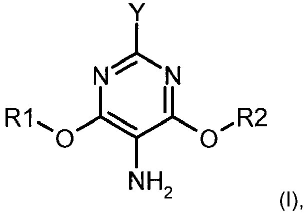 in der
- R¹, R²: unabhängig voneinander für ein, eine C₁-C₆-Alkylgruppe, eine Hydroxy-C₁-C₆-alkylgruppe, eine C₁-C₆-Alkoxy-C₁-C₆-alkylgruppe, eine C₁-C₆-Alkanoylgruppe, eine C₁-C₆-Alkoxycarbonylgruppe, oder eine Arylgruppe, stehen,
- Y: für eine Hydroxygruppe, eine Aminogruppe oder eine C₁-C₆-Alkylaminogruppe steht, und/oder deren physiologisch verträgliches Salz.

Unter keratinischen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose verwendet werden.

Die erfindungsgemäßen Mittel enthalten die Verbindungen der Formel (I) in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Es ist aber auch denkbar, die Farbstoffvorprodukte gemäß Formel (I) in eine pulverförmige oder auch tablettenförmige Formulierung zu integrieren. Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Im Folgenden werden Beispiele für die in Formel (I) genannten Substituenten R1, R2, R3 und R4 exemplarisch genannt:
Beispiele für C₁-C₆-Alkylreste sind -CH₃, -CH₂CH₃, -CH₂CH₂CH₃,-CH(CH₃)₂, -CH₂CH₂CH₂CH₃, -CH₂CH(CH₃)₂, -CH(CH₃)CH₂CH₃, -C(CH₃)₃, -(CH₂)₄CH₃, -(CH₂)₅CH₃. Besonders bevorzugte Alkylreste sind Methyl und Ethyl. Beispiele für C₁-C₆-Hydroxyalkylgruppen sind -CH₂OH,-CH₂CH₂OH, -CH₂CH₂CH₂OH, -CH₂CH(OH)CH₃, -CH₂CH₂CH₂CH₂OH, wobei -CH₂CH₂OH bevorzugt ist. Beispielhaft für eine C₁-C₆-Alkoxy-C₁-C₆-alkylgruppe können die Gruppen H₃C-O-CH₂-, H₃C-O-CH₂-CH₂-, H₃C-O-CH₂-CH₂-CH₂-, H₃C-CH₂-O-CH₂-, H₃C-CH₂-O-CH₂-CH₂- und H₃C-CH₂-O-CH₂-CH₂-CH2- genannt werden, wobei die Gruppen H₃C-O-CH₂-CH₂- und H₃C-CH₂-O-CH₂-CH₂- bevorzugt sind. Eine besonders bevorzugte eine C₁-C₆-Alkanoylgruppe ist die Acetylgruppe (H₂C-C(O)-). Beispielhaft für eine C₁-C₆-Alkoxycarbonylgruppe können die Gruppen H₃C-O-C(O)- und H₃C-CH₂-O-C(O)- genannt werden. Als Beispiel für eine Arylgruppe kann vor allem die Phenylgruppe genannt werden.

Innerhalb der Gruppe der erfindungsgemäßen tetrabsubstituierten Pyrimidinderivate zeigen bestimmte Substitionsmuster besonders vorteilhafte Eigenschaften. Verbindungen der Formel (I), bei welchen die Reste R1 und/oder R2 unabhängig voneinander mit einer C₁-C₆-Alkylgruppe, eine Hydroxy-C₁-C₆-alkylgruppe, eine C₁-C₆-alkoxy-C₁-C₆-alkylgruppe, eine C₁-C₆-Alkanoylgruppe, eine C₁-C₆-Alkoxycarbonylgruppe oder eine Arylgruppe stehen, ziehen gut auf die Keratinfaser auf. Inneralb dieser Ausführungsform ist es ganz besonders bevorzugt, wenn R1 und R2 jeweils unabhängig voneinander für eine C₁-C₆-Alkylgruppe stehen, da bei entsprechend substituierten Verbindungen der Formel (I) die höchste Farbintensität zu beobachten ist.

Ein besonders bevorzugtes Mittel zur oxidativen Färbung keratinischer Fasern ist daher dadurch gekennzeichnet, dass es mindestens eine Verbindung der Formel (I) enthält, in der R¹ und R² unabhängig voneinander für eine C₁-C₆-Alkylgruppe, stehen.

Im Zuge der zu dieser Erfindung führenden Arbeiten hat sich weiterhin herausgestellt, dass Verbindungen der Formel (I) ganz besonders gute anwendungstechnische Eigenschaften besitzen, wenn der Rest Y für eine Hydroxygruppe oder für eine Aminogruppe steht.

Ein besonders bevorzugtes Mittel zur oxidativen Färbung keratinischer Fasern ist weiterhin dadurch gekennzeichnet, dass es mindestens eine Verbindung der Formel (I) enthält, in der Y für eine Hydroxygruppe oder für eine Aminogruppe steht.

Besonders bevorzugt ist demnach ein Mittel zur oxidativen Färbung keratinischer Fasern, enthaltend in einem kosmetischen Träger als Oxidationsfarbstoffvorprodukt vom Entwickler-Typ mindestens eine Verbindung der Formel (I), 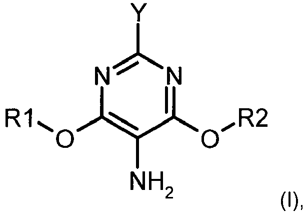 in der
- R¹, R²: unabhängig voneinander für eine C₁-C₆-Alkylgruppe stehen,
- Y: für eine Hydroxygruppe oder eine Aminogruppe steht,
und/oder deren physiologisch verträgliches Salz.

Bei Untersuchung der auf Keratinfasern ausgefärbten Mittel hinsichtlich ihrer Echtheitseigenschaften zeigte sich, dass die beste Waschechtheit und die beste Lichtechtheit dann erzielt werden kann, wenn zur Färbung eine Verbindung der Formel (I) eingesetzt wird, bei welcher der Rest R1 für eine Methylgruppe oder für eine Ethylgruppe steht, der Rest R2 für eine Methylgruppe oder für eine Ethylgruppe steht und der Rest Y für eine Aminogruppe steht.

Ein explizit ganz besonders bevorzugtes Mittel zur oxidativen Färbung keratinischer Fasern ist daher dadurch gekennzeichnet, dass R¹ und R² beide für eine Methylgruppe stehen und Y für eine Aminogruppe steht.

Ein explizit ganz besonders bevorzugtes Mittel zur oxidativen Färbung keratinischer Fasern ist auch dadurch gekennzeichnet, dass R¹ und R² beide für eine Ethylgruppe stehen und Y für eine Aminogruppe steht.

Unter Berücksichtigung der zuvor genannten Ausführungsformen ist demnach ein Mittel zum oxidativen Färben von keratinischen Fasern bevorzugt, welches mindestens eine der nachfolgenden Verbindungen der Formel (I) enthält:
- 4,6-Dimethoxypyrimidin-2,5-diamin 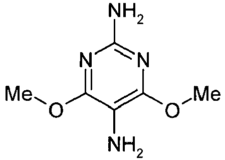
- 4-Methoxy-6-ethoxypyrimidin-2,5-diamin 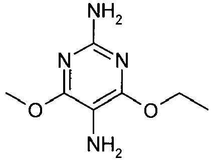
- 4-Methoxy-6-propoxypyrimidin-2,5-diamin 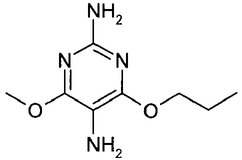
- 4-Methoxy-6-(2-methylpropoxy)pyrimidin-2,5-diamin 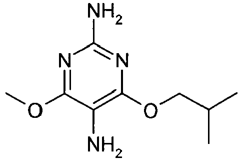
- 4,6-Diethoxypyrimidin-2,5-diamin 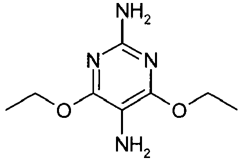
- 4-Ethoxy-6-propoxypyrimidin-2,5-diamin 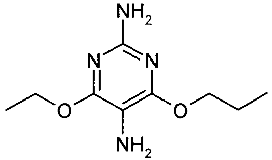
- 4-Ethoxy-6-(2-methylpropoxy)pyrimidin-2,5-diamin 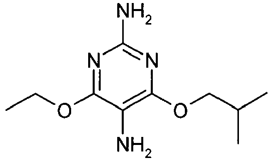
- [(2,5-Diamino-6-methoxypyrimidin-4-yl)oxy]methanol 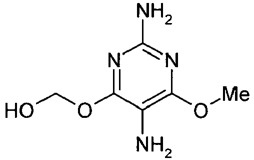
- [(2,5-Diamino-6-ethoxypyrimidin-4-yl)oxy]methanol 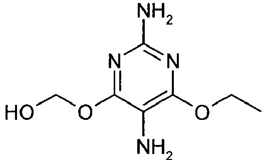
- [(2,5-Diamino-6-propoxypyrimidin-4-yl)oxy]methanol 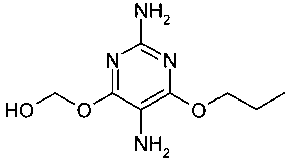
- [(2,5-Diamino-6-(2-methylpropoxy)pyrimidin-4-yl)oxy]methanol 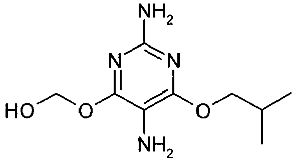
- {[2,5-Diamino-6-(hydroxymethoxy)pyrimidin-4-yl]oxy}methanol 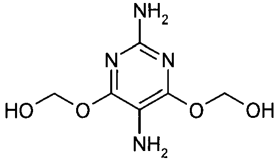
- [(2,5-Diamino-6-methoxypyrimidin-4-yl)oxy]ethanol 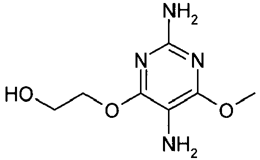
- [(2,5-Diamino-6-ethoxypyrimidin-4-yl)oxy]ethanol 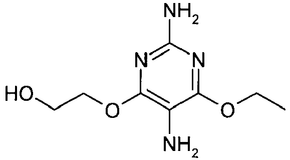
- [(2,5-Diamino-6-propoxypyrimidin-4-yl)oxy]ethanol 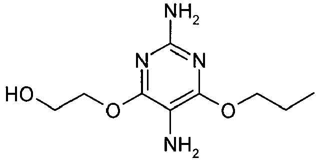
- [(2,5-Diamino-6-(2-methylpropoxy)pyrimidin-4-yl)oxy]ethanol 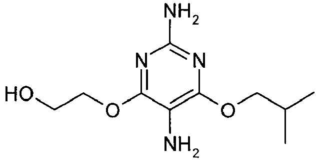
- 2-{[2,5-Diamino-6-(2-hydroxyethoxy)pyrimidin-4-yl]oxy}ethan-1-ol 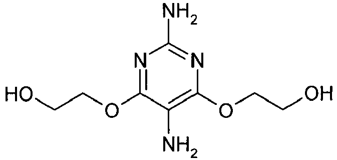
- [(2,5-Diamino-6-methoxypyrimidin-4-yl)oxy]propanol 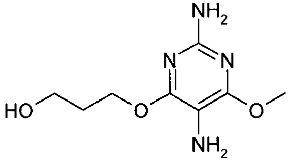
- [(2,5-Diamino-6-ethoxypyrimidin-4-yl)oxy]propanol 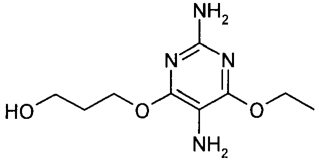
- [(2,5-Diamino-6-propoxypyrimidin-4-yl)oxy]propanol 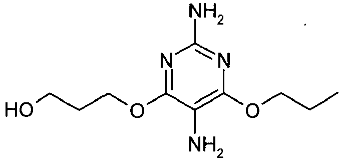
- [(2,5-Diamino-6-(2-methylpropoxy)pyrimidin-4-yl)oxy]propanol 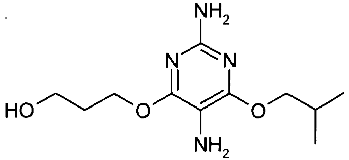
- [(2,5-Diamino-6-(2-hydroxypropoxy)pyrimidin-4-yl)oxy]propanol 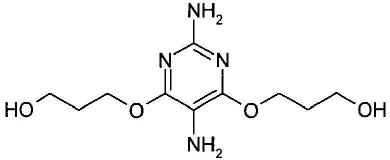
- 2,5-Diamino-6-methoxypyrimidin-4-yl acetat 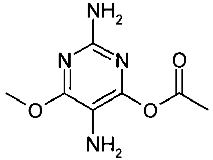
- 2,5-Diamino-6-ethoxypyrimidin-4-yl acetat 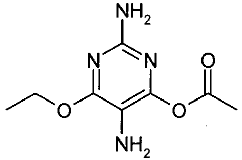
- 2,5-Diamino-6-propoxypyrimidin-4-yl acetat 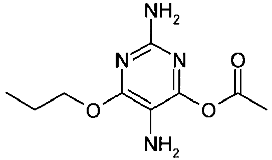
- 2,5-Diamino-6-(2-methylpropoxy)pyrimidin-4-yl acetat 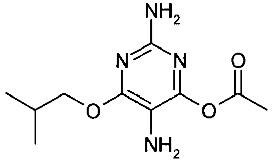
- 6-(Acetyloxy)-2,5-diaminopyrimidin-4-yl acetat 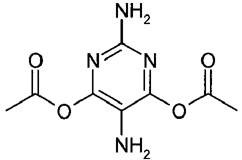
- 2,5-Diamino-6-methoxypyrimidin-4-yl propanoat 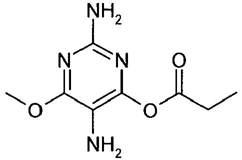
- 2,5-Diamino-6-ethoxypyrimidin-4-yl propanoat 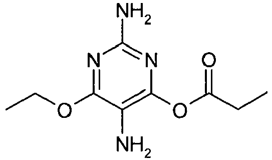
- 2,5-Diamino-6-propoxypyrimidin-4-yl propanoat 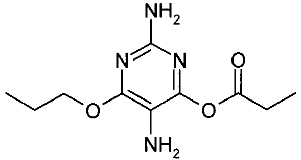
- 2,5-Diamino-6-(2-methylpropoxy)pyrimidin-4-yl propanoat 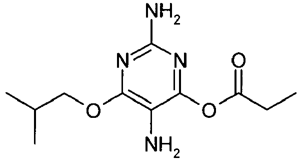
- 2,5-Diamino-6-(propanoyloxy)pyrimidin-4-yl propanoat 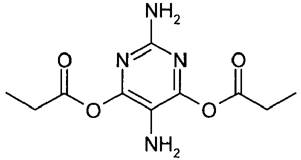
- 2,5-Diamino-6-methoxypyrimidin-4-yl butanoat 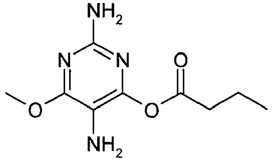
- 2,5-Diamino-6-ethoxypyrimidin-4-yl butanoat 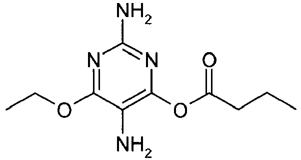
- 2,5-Diamino-6-propoxypyrimidin-4-yl butanoat 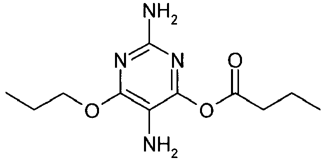
- 2,5-Diamino-6-(2-methylpropoxy)pyrimidin-4-yl butanoat 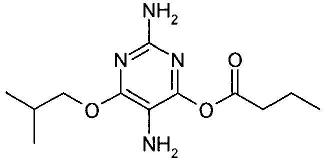
- 2,5-Diamino-6-(butanoyloxy)pyrimidin-4-yl butanoat 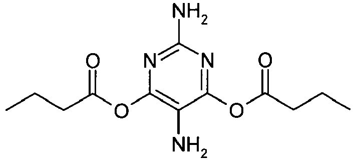
- 2,5-Diamino-6-methoxypyrimidin-4-yl methylcarbonat 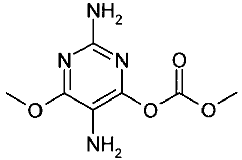
- 2,5-Diamino-6-ethoxypyrimidin-4-yl methylcarbonat 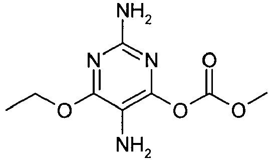
- 2,5-Diamino-6-propoxypyrimidin-4-yl methylcarbonat 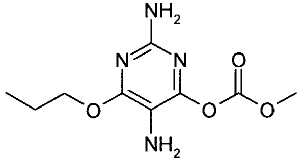
- 2,5-Diamino-6-(2-methylpropoxy)pyrimidin-4-yl methylcarbonat 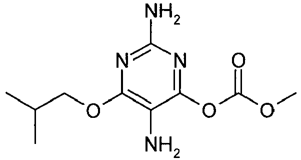
- 2,5-Diamino-6-methoxypyrimidin-4-yl ethylcarbonat 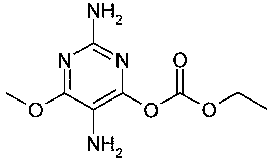
- 2,5-Diamino-6-ethoxypyrimidin-4-yl ethylcarbonat 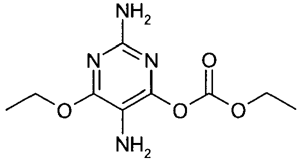
- 2,5-Diamino-6-propoxypyrimidin-4-yl ethylcarbonat 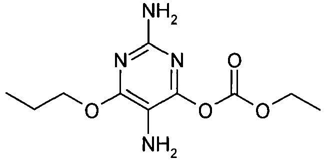
- 2,5-Diamino-6-(2-methylpropoxy)pyrimidin-4-yl ethylcarbonat 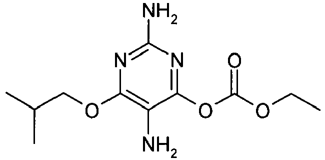
- 4-[(Diethylamino)ethoxy]-6-propoxypyrimidin-2,5-diamin
- 4-[(Diethylamino)ethoxy]-6-(2-methylpropoxy)pyrimidin-2,5-diamin
- 4-Methoxy-6-phenoxypyrimidin-2,5-diamin 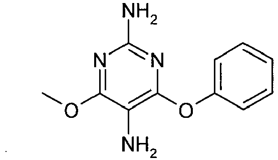
- 4-Ethoxy-6-phenoxypyrimidin-2,5-diamin 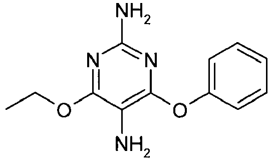
- 4-Propoxy-6-phenoxypyrimidin-2,5-diamin 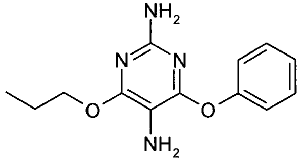
- 4-(2-Methylpropoxy)-6-phenoxypyrimidin-2,5-diamin 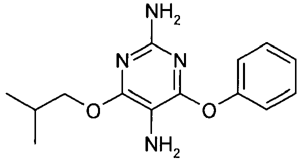
- 4,6-Diphenoxypyrimidin-2,5-diamin

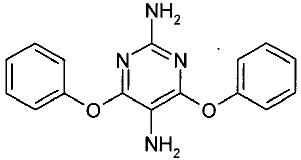

Innerhalb der vorgenannten Gruppe sind einige Verbindungen nochmals ganz besonders bevorzugt. Ein besonders bevorzugtes Mittel zur oxidativen Färbung keratinischer Fasern ist daher weiterhin dadurch gekennzeichnet, dass es mindestens eine Verbindung der Formel (I) enthält, die ausgewählt ist aus 4,6-Dimethoxypyrimidin-2,5-diamin, 4-Methoxy-6-ethoxypyrimidin-2,5-diamin, 4,6-Diethoxy-pyrimidin-2,5-diamin, [(2,5-Diamino-6-methoxypyrimidin-4-yl)oxy]methanol, [(2,5-Diamino-6-ethoxypyrimidin-4-yl)oxy]methanol, [(2,5-Diamino-6-methoxypyrimidin-4-yl)oxy]ethanol, [(2,5-Diamino-6-ethoxypyrimidin-4-yl)oxy]ethanol, und/oder deren physiologisch verträglichen Salzen.

Bei den erfindungsgemäßen Verbindungen der Formel (I) handelt es sich um Aminoverbindungen. Diese können auch in Form ihrer physiologisch verträglichen Salze, insbesondere der Chloride, der Sulfate und Bromide, eingesetzt werden. Weitere bevorzugte Salze sind von Sulfonsäuren abgeleitet, wie Benzolsulfonate, p-Toluolsulfonsulfonate, C₁-C₄-Alkansulfonate oder Trifluormethansulfonate. Abhängig von der Anzahl der in den erfindungsgemäßen Verbindungen enthaltenen Aminogruppen können Mono-, Di-, Tri-, Tetra- und höhere Addukte als Salze vorliegen.

Um eine ausreichend hohe Farbintensität auf den Keratinfasern erzielen zu können, ist es bevorzugt, die erfindungemäßen Verbindungen der Formel (I) in bestimmten Mengenbereichen im oxidativen Färbemittel einzusetzen.

Ein erfindungsgemäß bevorzugtes Mittel ist dadurch gekennzeichnet, dass es eine oder mehrere Verbindungen der Formel (I) in einer Gesamtmenge von 0,001 bis 5,0 Gew.-%, bevorzugt von 0,025 bis 2,5 Gew.-%, besonders bevorzugt von 0,05 bis 2,0 Gew.-% und insbesondere bevorzugt von 0,1 bis 1,5 Gew.-%, jeweils bezogen auf des Gesamtgewicht des anwendungsbereiten Mittels, enthält. Alle vorgenannten Gewichtsmengen sind herbei auf das Gesamtgewicht des anwendungbereiten Mittels bezogen. Bei dem anwendungsbereiten Mittel handelt es sich um das Mittel, das zur Anwendung bereit ist und vom Anwender oder Friseur direkt auf das Haar appliziert werden kann. Bei den erfindungsgemäßen Mitteln handelt es sich um Mittel zum oxidativen Färben von Keratinfasern - diese benötigen zur Ausbildung der Farbstoffe ein Oxidationsmittel (bevorzugt Wasserstoffperoxid). Aus Stabiliätsgründen und zur Vermeidung einer vorzeitigen Farbstoffbildungsreaktion werden das Mittel, welches die Oxidationsfarbstoffvorprodukte enthält (M1), und das Oxidationsmittel (M2) in getrennten Containern bereitgestellt. Kurz vor der Anwendung werden dann beide Mittel (M1) und (M2) vermischt, wodurch das anwendungsbereite Färbemittel erhalten wird. Relevant für die auf den Keratinfasern erzielbare Farbintensität ist der Gehalt der Oxidationsfarbstoffe im anwendungsbereiten Mittel. Daher ist es notwendig, alle Mengenangaben, die den Gehalt der Verbindungen der Formel (I) beziffern, auf das Gewicht des anwendungsbereiten Mittels zu beziehen.

Grundsätzlich können die Verbindungen der Formel (I) als alleinige farbverändernde Verbindungen im erfindungsgemäßen Mittel enthalten sein. Das Prinzip der oxidativen Färbung beruht jedoch darauf, dass die Oxidationsfarbstoffvorprodukte vom Entwicklertyp erst durch die gezielte Kupplungsreaktion mit weiteren Oxidationsfarbstoffvprodukten vom Kupplertyp die eigentlichen Farbstoffe ausbilden. Abhängig vom dem bzw. den eingesetzten Kupplern können hierbei verschiedene Nuancen erzielt werden. Es ist daher erfindungsgemäß bevorzugt, die Verbindung(en) der Formel (I) in Kombination mit einem oder mehreren Oxidationsfarbstoffen vom Kupplertyp im erfindungsgemäßen Mittel einzusetzen.

Kupplerkomponenten bilden im Rahmen der oxidativen Färbung allein keine signifikante Färbung aus, sondern benötigen stets die Gegenwart von Entwicklerkomponenten. Kupplerkomponenten im Sinne der Erfindung erlauben mindestens eine Substitution eines chemischen Restes des Kupplers durch die oxidierte Form der Entwicklerkomponente. Dabei bilden sich kovalente Bindungen zwischen Kuppler- und Entwicklerkomponente aus.

Als erfindungsgemäß geeignete Kupplerkomponente wird bevorzugt mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate, die von Formel (I) verschieden und nicht tetrasubstituiert sind,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin.

Gemische aus zwei oder mehreren Verbindungen aus einer oder mehreren dieser Klassen sind im Rahmen dieser Ausführungsform ebenso erfindungsgemäß.

Geeignete Kuppler können ausgewählt werden aus der Gruppe aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)-propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]-ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxy-pyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin und/oder 7-Hydroxyindolin und deren physiologisch verträglichen Salzen.

In diesem Zusammenhang hat sich gezeigt, dass durch die Wahl spezifischer Entwickler/Kuppler-Kombinationen auch die Echtheitseigenschaften der resultierenden Färbungen stark beeinflusst werden können. Bei bestimmten Kombinationen von Entwicklern mit speziellen Kupplern sind die Waschechtheiten und die Lichtechtheiten demnach besonders gut.

Ein ganz besonders bevorzugtes Mittel ist daher dadurch gekennzeichnet, dass es zusätzlich mindestens ein Oxidationsfarbstoffvorprodukt vom Kupplertyp aus der Gruppe 3-Aminophenol, 5-Amino-2-methylphenol, 3-Amino-2-chlor-6-methylphenol, 2-(2,4-Diaminophenoxy)ethanol, 1,3-Bis-(2,4-diaminophenoxy)propan, 1-Methoxy-2-amino-4-(2-hydroxyethylamino)benzol, 2,7- Dihydroxynaphthalin, 1,5- Dihydroxynaphthalin, 1-Naphthol, Resorcin, 2-Methylresorcin, 4-Chlorresorcin, 2-Amino-3-hydroxypyridin, 3-Amino-6-methoxy-2-methylaminopyridin, 2,6-Dihydroxy-3,4-dimethylpyridin, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-hydroxyethyl)amino]-2-methylphenyl}amino)ethanol, 2,6-Dihydroxy-3,4-dimethylpyridin 1-Methyl-2,6-bis(2-hydroxyethyl-amino)benzol und/oder einem physiologisch verträglichen Salz dieser Verbindungen enthält.

Für die Entwicklung einer marktreifen, allen anwendungstechnischen Ansprüchen genügenden Nuance ist es in den meisten Fällen nicht ausreichend, einen Entwickler in Kombination mit einem Kuppler einzusetzen. Insbesondere für die farbliche Feinabstimmung werden in den vermarkteten Produkten Farbstoffkombinationen mit mehreren verschiedenen Oxidationsfarbstoffvorprodukten eingesetzt.

Bei der Nuancenentwicklung mit den erfindungsgemäßen Verbindungen der Formel (I) wurde gefunden, dass bestimmte Kombinationen aus Entwickler der Formel (I) / Kuppler 1 / Kuppler 2 zu Färbungen mit besonders herausragenden Intensitäten und Echtheitseigenschaften führen.

Ein ganz besonders bevorzugtes Mittel ist daher dadurch gekennzeichnet, dass es zusätzlich
- ein oder mehrere Oxidationsfarbstoffvorprodukte vom Kuppertyp aus der Gruppe Resorcin, 2-Methylresorcin und/oder 4-Chlorresorcin enthält und
- ein oder mehrere Oxidationsfarbstoffvorprodukte vom Kuppertyp aus der Gruppe (2,4-Diaminophenoxy)ethanol, 1,3-Bis(2,4-diaminophenoxy)propan, 1-Methoxy-2-amino-4-(2-hydroxyethylamino)benzol enthält.

Ein ganz besonders bevorzugtes Mittel ist daher weiterhin dadurch gekennzeichnet, dass es zusätzlich
- ein oder mehrere Oxidationsfarbstoffvorprodukte vom Kuppertyp aus der Gruppe Resorcin, 2-Methylresorcin und/oder 4-Chlorresorcin enthält und
- ein oder mehrere Oxidationsfarbstoffvorprodukte vom Kuppertyp aus der Gruppe 2-Amino-3-hydroxypyridin, 3-Amino-6-methoxy-2-methylaminopyridin und/oder 2,6-Dihydroxy-3,4-dimethylpyridin enthält.

Ein ganz besonders bevorzugtes Mittel ist daher weiterhin dadurch gekennzeichnet, dass es zusätzlich
- ein oder mehrere Oxidationsfarbstoffvorprodukte vom Kuppertyp aus der Gruppe Resorcin, 2-Methylresorcin und/oder 4-Chlorresorcin enthält und
- ein oder mehrere Oxidationsfarbstoffvorprodukte vom Kuppertyp aus der Gruppe 1-Naphthol, 1,5-Dihydroxynaphthalin und/oder 1-Methyl-2,6-bis(2-hydroxyethylamino)benzol enthält.

Die Kupplerkomponenten werden bevorzugt in einer Gesamtmenge von 0,0001 bis 10 Gew.-%, vorzugsweise 0,01 bis 5,0 Gew.-%, jeweils bezogen auf das anwendungsbereite Mittel, verwendet. Neben der bzw. den Verbindungen der Formel (I) kann das erfindungsgemäße Mittel auch noch weitere Oxidationsfarbstoffvorprodukte vom Entwicklertyp enthalten.

Bevorzugte weitere Entwicklerkomponenten werden ausgewählt aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetra-oxadecan, 4-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-tri-aminopyrimidin, sowie den physiologisch verträglichen Salzen dieser Verbindungen. Besonders bevorzugte zusätzliche Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin und/oder 4,5-Diamino-1-(2-hydroxyethyl)-pyrazol sowie deren physiologisch verträglichen Salze.

Ein besonders bevorzugtes Mittel ist daher weiterhin dadurch gekennzeichnet, dass es zusätzlich mindestens ein Oxidationsfarbstoffvorprodukt vom Entwicklertyp aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diaza-cycloheptan, 1,10-Bis-(2,5-diaminophenyl)-1,4,7,10-tetraoxadecan, 4-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin benzol und/oder einem physiologisch verträglichen Salz dieser Verbindungen enthält.

Innerhalb der Gruppe der zusätzlich im Mittel einsetzbaren Entwickler sind ausgesuchte Verbindungen besonders gut mit den Verbindungen der Formel (I) kompatibel. Hierbei handelt es sich um die Oxidationsfarbstoffe vom Entwicklertyp, die aus der Gruppe 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, 4,5-Diamino-1-(2-hydroxyethyl)-pyrazol und/oder einem physiologisch verträglichen Salz hiervon ausgewählt sind. Der Einsatz dieser Kombinationen bietet den Vorteil, dass die entsprechenden Färbemittel besonders geringe Hautirritationen hervorrufen und keine Nuancenverschiebungen auf den Keratinfasern auftreten. Ein ganz besonders bevorzugtes Mittel ist daher weiterhin dadurch gekennzeichnet, dass es zusätzlich mindestens ein Oxidationsfarbstoffvorprodukt vom Entwicklertyp aus der Gruppe 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, 4,5-Diamino-1-(2-hydroxyethyl)pyrazol und/oder einem physiologisch verträglichen Salz dieser Verbindungen enthält.

Die zusätzlichen Entwicklerkomponenten werden bevorzugt in einer Gesamtmenge von 0,0001 bis 10 Gew.-%, vorzugsweise 0,001 bis 5 Gew.-%, jeweils bezogen auf das anwendungsbereite Mittel, verwendet.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe können in einer Gesamtmenge von 0,001 bis 20 Gew.-%, insbesondere von 0,05 bis 5 Gew.-%, jeweils bezogen auf das Gewicht des anwendungsbereiten Mittels, eingesetzt werden Die Gesamtmenge an direktziehenden Farbstoffen im anwendungsbereiten Mittel beträgt vorzugsweise höchstens 3 Gew.-%.

Direktziehende Farbstoffe können in anionische, kationische und nichtionische direktziehende Farbstoffe unterteilt werden, die entsprechend der Anforderungen der Trägerbasis vom Fachmann ausgewählt und eingesetzt werden.

Bevorzugte anionische direktziehende Farbstoffe sind die unter den internationalen Bezeichnungen bzw. Handelsnamen Bromphenolblau, Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Die erfindungsgemäßen Mittel können neben der Verbindung gemäß Formel (I) auch naturanaloge Farbstoffe enthalten. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders gut als Vorstufen naturanaloger Haarfarbstoffe geeignet sind Derivate des 5,6-Dihydroxyindolins, insbesondere 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure, sowie weiterhin Derivate des 5,6-Dihydroxyindols, insbesondere 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, sowie physiologisch verträgliche Salze der vorstehend genannten Verbindungen.

Die Farbstoffvorstufen naturanaloger Farbstoffe werden jeweils bevorzugt in einer Menge von 0,001 bis 5 Gew.-%, bezogen auf das Gewicht des anwendungsbereiten Mittels, eingesetzt.

Die Farbstoffbildung wird durch die Anwesenheit eines Oxidationsmittels initiiert, wobei das Oxidationsmittel gleichzeitig auch die haareigenen Farbstoffe (Melanine) oxidativ zerstört und auf diese Weise eine Aufhellung der Ursprungshaarfarbe bewirkt. Die Einsatzkonzentration des Oxidationsmittels wird hierbei abhängig von dem Aufhelleffekt gewählt, der zusätzlich zur Färbung der Keratinfasern gewünscht ist. Als Oxidationsmittel kommen Persulfate, Peroxodisulfate, Chlorite, Hypochlorite und insbesondere Wasserstoffperoxid und/oder eines seiner festen Anlagerungsprodukte an organische oder anorganische Verbindungen in Frage.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht. Für den Anwender des oxidativen Färbemittels ist es besonders komfortabel und daher bevorzugt, die Mittel zu verwenden, die ihm in einer entsprechenden Mehrkomponenten-Verpackungseinheit (Kit-of-parts) zur Verfügung gestellt werden.

Ein zweiter Gegenstand der vorliegenden Erfindung ist eine Mehrkomponenten-Verpackungseinheit (Kit-of-parts) zum oxidativen Färben von keratinischen Fasern, umfassend zwei getrennt voneinander verpackte Mittel (M1) und (M2), wobei
- es sich bei dem Mittel (M1) um ein Mittel des ersten Erfindungsgegenstands handelt
- das Mittel (M2) in einem wässrigen kosmetischen Träger ein Oxidationsmittel enthält.

Bevorzugt enthält die Oxidationsmittelzubereitung (d.h. das Mittel (M2)) als Oxidationsmittel Wasserstoffperoxid und/oder eines seiner festen Anlagerungsprodukte an organische oder anorganische Verbindungen, wie Harnstoff, Melamin sowie Natriumborat.

Bevorzugt beträgt die Menge an Oxidationsmittel im anwendungsbereiten Mittel (d.h. im Gemisch aus (M1) und (M2)) 0,5 bis 12 Gew.-%, bevorzugt 0,5 bis 6 Gew.-%, weiter bevorzugt von 1,0 bis 5,0 Gew.-%, noch weiter bevorzugt von 1,5 bis 4,0 Gew.-% und besonders bevorzugt von 1,5 bis 3,0 Gew.-% - bezogen auf das Gesamtgewicht der Gemisches aus (M1) und (M2).

Bevorzugt ist daher eine Mehrkomponenten-Verpackungseinheit (Kit-of-parts) zum oxidativen Färben von keratinischen Fasern, umfassend zwei getrennt voneinander verpackte Mittel (M1) und (M2), wobei
- es sich bei dem Mittel (M1) um ein Mittel des ersten Erfindungsgegenstands handelt
- das Mittel (M2) in einem wässrigen kosmetischen Träger Wasserstoffperoxid enthält und
- die Mischung aus (M1) und (M2) Wasserstoffperoxid in einer Menge von 0,5 bis 6,0 Gew.-%, bevorzugt von 1,0 bis 5,0 Gew.-%, weiter bevorzugt von 1,5 bis 4,0 Gew.-% und besonders bevorzugt von 1,5 bis 3,0 Gew.-% - bezogen auf das Gesamtgewicht der Gemisches aus (M1) und (M2) - enthält.

Erfindungsgemäß kann aber das Oxidationsfärbemittel auch zusammen mit einem Katalysator auf das Haar aufgebracht werden, der die Oxidation der Farbstoffvorprodukte aktiviert. Solche Katalysatoren sind z. B. bestimmte Enzyme, lodide, Chinone oder Metallionen.

Weiterhin hat es sich als vorteilhaft erweisen, wenn die Oxidationsmittelzubereitungen mindestens einen Stabilisator oder Komplexbildner enthalten. Gebräuchliche und im Rahmen der vorliegenden Erfindung bevorzugte Komplexbildner und Stabilisatoren sind beispielsweise Polyoxycarbonsäuren, Polyamine, Ethylendiamintetraessigsäure (EDTA), N-Hydroxyethylethylendiamintriessigsäure, Diethylentriaminpentaessigsäure (DTPA), Ethylendiamindibernsteinsäure (EDDS), Hydroxyethyliminodiessigsäure, Nitridodiessigsäure-3-propionsäure, Isoserindiessigsäure, N,N-Di-(2-hydroxyethyl)glycin, N-(1,2-Dicarboxy-2-hydroxyethyl)glycin, N-(1,2-Dicarboxy-2-hydroxyethyl)asparaginsäure oder Nitrilotriessigsäure (NTA), Ethylendiamindiglutarsäure (EDGA), 2-Hydroxypropylendiamindibernsteinsäure (HPDS), Glycinamid-N,N'-dibernsteinsäure (GADS), Ethylendiamin-N-N'-diglutarsäure (EDDG), 2-Hydroxypropylendiamin-N-N'-dibernsteinsäure (HPDDS), Diaminoalkyldi-(sulfobernsteinsäure) (DDS), Ethylendicysteinsäure (EDC), Ethylendiamin-N-N'-bis(ortho-hydroxyphenyl)essigsäure (EDDHA), N-2-Hydroxyethylamin-N,N-diessigsäure, Glyceryliminodiessigsäure, Iminodiessigsäure-N-2-hydroxypropylsulfonsäure, Asparaginsäure-N-carboxymethyl-N-2,5-hydroxypropyl-3-sulfonsäure,β-Alanin-N,N'-diessigsäure, Asparaginsäure-N,N'-diessigsäure, Asparaginsäure-N-monoessigsäure, Dipicolinsäure, sowie deren Salze und/oder Derivate, geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppen-haltige Derivate, Aminophosphonsäuren wie Ethylendiamintetra(methylenphosphonsäure) (EDTMP), Diethylentriaminpenta(methylenphosphonsäure) (DTPMP) sowie deren höhere Homologe, oder Nitrilo-tri(methylenphosphonsäure), Phosphonopolycarbonsäuren wie 2-Phosphonobutan-1,2,4-tricarbonsäure, Cyclodextrine, sowie Alkalistannate (Natriumstannat), Alkalipyrophosphate (Tetranatriumpyrophosphat, Dinatriumpyrophosphat), Alkaliphosphate (Natriumphosphat), und Phosphorsäure sowie deren Salze.

Für eine ausreichende Quellung der Keratinfasern ist das anwendungsbereite oxidative Färbemittel bevorzugt auf einen alkalischen pH-Wert eingestellt. Auch die Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn das anwendungsbereiten Mittels einen pH-Wert von 7,0 bis 10,5, bevorzugt von 7,5 bis 9,5, weiter bevorzugt von 8,0 bis 9,0 besitzt.

Der Wassergehalt in der Anwendungmischung liegt bevorzugt bei einer Menge von mind. 50 Gew.-%, bevorzugt von mind. 60 Gew.-%, weiter bevorzugt von mind. 70 Gew.-% und besonders bevorzugt von mind. 80 Gew.-% - bezogen auf das Gesamtgewicht der Gemisches aus (M1) und (M2).

Eine besonders bevorzugte Mehrkomponenten-Verpackungseinheit (Kit-of-parts) zum oxidativen Färben von keratinischen Fasern, ist dadurch weiterhin gekennzeichnet, dass
- die Mischung aus (M1) und (M2) Wasser in einer Menge von mind. 50 Gew.-%, bevorzugt von mind. 60 Gew.-%, weiter bevorzugt von mind. 70 Gew.-% und besonders bevorzugt von mind. 80 Gew.-% - bezogen auf das Gesamtgewicht der Gemisches aus (M1) und (M2) - enthält und
- die Mischung aus (M1) und (M2) einen pH-Wert von 7,0 bis 10,5, bevorzugt von 7,5 bis 9,5, weiter bevorzugt von 8,0 bis 9,0 besitzt.

Bei den angegebenen pH-Werten handelt es sich um Werte, die bei einer Temperatur von 22 °C gemessen wurden. Die Messung des pH-Wertes kann unter Zuhilfenahme einer handelsüblichen Glaselektrode gemessen werden - Glaselektroden sind oft als Einstabmessketten konzipiert.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)-Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methyl-propanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen. Bevorzugt werden das oder die Alkalisierungsmittel zusammen mit den Oxidationsfarbstoffvorprodukten in der Färbezubereitung (M1) konfektioniert.

Die oxidativen Farbänderungsmittel (d.h. die Färbemittel (M1)) und/oder die Oxidationsmittelzubereitung (d.h. die Mittel (M2)) können weiterhin zusätzliche Wirk-, Hilfs- und Zusatzstoffe enthalten, um die Färbe- bzw. Aufhellleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher gegebenenfalls zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt. Erfindungsgemäß geeignete Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Erfindungsgemäß geeignete Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß geeignete Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die anionischen, nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Die anwendungsbereiten Farbveränderungsmittel können auch mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Geeignete Verdickungsmittel sind anionische, synthetische Polymere, kationische, synthetische Polymere, natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen, nichtionische, vollsynthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazoliniummethochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, jeweils bezogen auf das Gesamtgewicht der Färbezubereitung (K1) und/oder der Oxidationsmittelzubereitung (K2), eingesetzt.

Die erfindungsgemäßen Mittel zeigen eine außerordentlich gute Eignung zur Färbung von keratinischen Fasern, wobei die gefärbten Fasern sehr gute Waschechtheiten, sehr gute Lichtechtheiten und ein sehr gutes Egalisiervermögen aufweisen.

Auch die erfindungsgemäßen Mehrkomponenten-Verpackungseinheiten (Kit-of-Parts) zeigen sehr gute Eignung, Färbungen mit sehr guten Waschechtheiten, guten Lichtechtheiten und gutem Egalisiervermögen zu erzielen.

Bezüglich weiterer bevorzugter Ausführungsformen des erfindungsgemäßen Mehrkomponenten-Verpackungseinheiten (Kit-of-Parts) und der erfindungsgemäßen Verwendungen gilt mutatis mutandis das zu den erfindungsgemäßen Mitteln Gesagte.

### Beispiele

### Synthesebeispiele

### Synthesebeispiel 1: Synthese von 4,6-Dimethoxypyrimidin-2,5-diamin, Dihydrochlorid (E1)

### 1.1. Synthese von 2-Amino-4,6-dimethoxypyrimidin

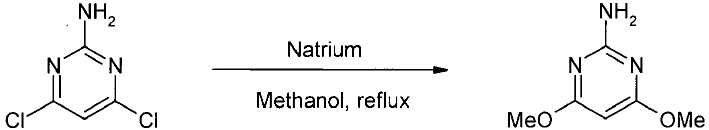

Zu einer Lösung von 15.0 g (0.652 mol) Natrium in 400 ml Methanol wurden 32.8 g (0.200 mol) 2-Amino-4,6-dichlorpyrimidin gegeben und unter Rühren für 13 Stunden unter Rückfluss erhitzt. Danach wurde für weitere 3 Tage bei Raumtemperatur gerührt. Nach Beendigung der Reaktion wurde das ausgefallene Natriumchlorid durch Filtration abgetrennt und die Lösung zur Trockene eingeengt. Der Rückstand wurde mit 500 ml Wasser gewaschen. Nach dem Trocknen erhielt man 2-Amino-4,6-dimethoxypyrimidin (26.5 g, 85%) als blassgelben Feststoff.
Smp.: 96 - 101°C
¹H-NMR (300 MHz, d₆-DMSO): δ = 3.80 (s, 6 H, 4-OMe, 6-OMe), 5.38 (s, 1 H, 5-H), 6.59 (s, 2 H, NH₂)
¹³C-NMR (125 MHz, d₆-DMSO): δ = 53.2 (4-OMe, 6-OMe), 77.9 (5-C), 162.8 (2-C), 171.7 (6-C, 4-C)

### 1.2. Synthese von 2-Amino-4,6-dimethoxy-5-nitrosopyrimidin

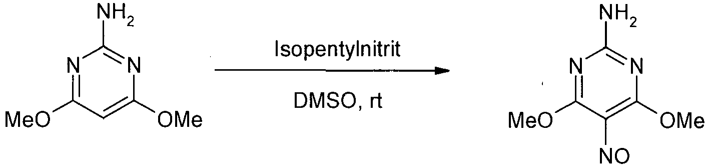

Zu einer Lösung von 41.9 g (0.270 mol) 2-Amino-4,6-dimethoxypyrimidin in 675 ml Dimethylsulfoxid wurden 34.8 g (0.297 mol) Isopentylnitrit gegeben und für 96 Stunden bei Raumtemperatur gerührt. Nach Beendigung der Reaktion wurde die tiefblaue Lösung auf 2.5 I Wasser gegossen und für 1 Stunde bei Raumtemperatur gerührt. Der entstandene Niederschlag wurde abfiltriert und mit Wasser gewaschen. Nach dem Trocknen erhielt man 2-Amino-4,6-dimethoxy-5-nitrosopyrimidin (32.8 g, 66%) als hellblauen Feststoff.
Smp.: 215 - 218°C (Zers.)
¹H-NMR (300 MHz, d₆-DMSO): δ = 3.94 (s, 6 H, 4-OMe, 6-OMe), 8.28 (s, 2 H, NH₂).
¹³C-NMR (125 MHz, d₆-DMSO): δ = 54.9 (4-OMe, 6-OMe), 141.9 (5-C), 163.3 (6-C, 4-C), 173-0 (2-C)

### 1.3. Synthese von 4,6-Dimethoxypyrimidin-2,5-diamin Dihydrochlorid (E1)

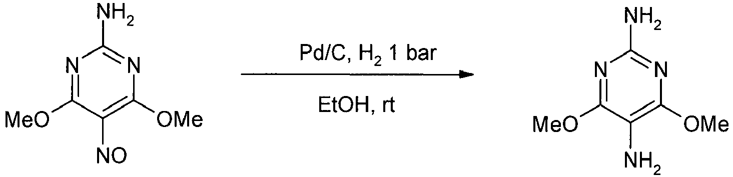

Zu einer Lösung von 7.37 g (0.04 mol) 2-Amino-4,6-dimethoxypyrimidin in 400 ml Ethanol wurden 0.5 g (5%) Palladium auf Kohle gegeben und für 17 Stunden unter einem Wasserstoffdruck von 1 bar bei Raumtemperatur geschüttelt. Nach Beendigung der Reaktion wurde die Reaktionsösung auf 60 ml Salzsäure (10% in Wasser) gegossen. Der Katalysator wurde abfiltriert. Das Filtrat wurde vollständig am Rotationsverdampfer eingeengt. Der Rückstand wurde in wenig eiskaltem Ethanol gerührt, hierbei entstand ein Niederschlag, der abfiltriert und mit wenig eiskaltem Ethanol nachgewaschen wurde. Nach dem Trocknen erhielt man 4,6-Dimethoxypyrimidin-2,5-diamin Dihydrochlorid (4.15 g, 50%) als hellbeigen Feststoff.
¹H-NMR (300 MHz, d₆-DMSO): δ = 3.98 (s, 6 H, 4-OMe, 6-OMe), 4.85 (brs, 4 H, NH₂)
¹³C-NMR (125 MHz, d₆-DMSO): δ = 58.0 (4-OMe, 6-OMe), 91.2 (5-C), 157.4 (2-C), 163.7 (4-C), 168.1 (6-C)

### Synthesebeispiel 2: Synthese von 4,6-Diethoxypyrimidin-2,5-diamin, Dihydrochlorid (E2)

### 2.1. Synthese von 2-Amino-4,6-diethoxypyrimidin

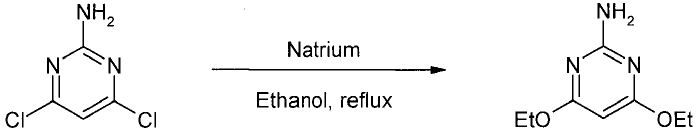

Zu einer Lösung von 15.3 g (0.670 mol) Natrium in 600 ml Ethanol wurde eine Suspension von 32.8 g (0.200 mol) 2-Amino-4,6-dichlorpyrimidin in 1.5 I Ethanol gegeben und unter Rühren für 6.5 Stunden unter Rückfluss erhitzt sowie 17 Stunden bei 70°C gerührt. Nach Beendigung der Reaktion wurde das ausgefallene NaCl durch Filtration abgetrennt und das Filtrat vollständig am Rotationsverdampfer eingeengt. Der Rückstand wurde mit 500 ml Wasser gewaschen. Nach dem Trocknen erhielt man 2-Amino-4,6-diethoxypyrimidin (35.7 g, 97%) als blassgelben Feststoff.
Smp.: 100 - 103°C
¹H-NMR (300 MHz, d₆-DMSO): δ = 1.21 (t, 6 H, CH₃), 4.21 (q, 4 H, CH₂), 5.30 (s, 1 H, 5-H), 6.49 (s, 2 H, NH₂)
¹³C-NMR (125 MHz, d₆-DMSO): δ = 14.5 (CH₃), 61.1 (-O-CH₂-), 78.3 (5-C), 162.8 (2-C), 171.2 (6-C, 4-C)

### 2.2. Synthese von 2-Amino-4,6-diethoxy-5-nitrosopyrimidin

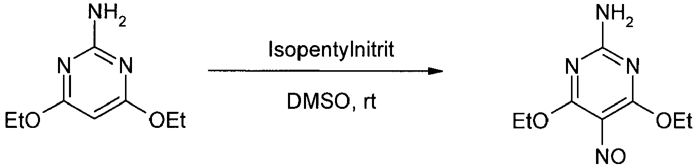

Zu einer Lösung von 34.8 g (0.19 mol) 2-Amino-4,6-diethoxypyrimidin in 475 ml Dimethylsulfoxid wurden 24.6 g (0.21 mol) Isopentylnitrit gegeben und für 119 Stunden bei Raumtemperatur gerührt. Nach Beendigung der Reaktion wurde die tiefblaue Lösung auf 1.9 l Wasser gegossen, 1 Stunde bei Raumtemperatur gerührt und der entstandene Niederschlag abfiltriert. Der Rückstand wurde mit Wasser gewaschen. Der erhaltene Feststoff wurde zweimal aus Ethylacetat umkristallisiert (600 ml und 300 ml). Nach dem Trocknen erhielt man 2-Amino-4,6-diethoxy-5-nitrosopyrimidin (10.8 g, 27%) als dunkelblauen Feststoff.
Smp.: 215 - 218°C (Zers.)
¹H-NMR (300 MHz, d₆-DMSO): δ = 1.38 (t, 6 H, CH₃), 4.47 (q, 4 H, CH₂), 8.21 (s, 2 H, NH₂) ¹³C-NMR (125 MHz, d₆-DMSO): δ = 14.3 (CH₃), 62.9 (-O-CH₂-), 141.4 (5-C), 163.0 (6-C, 4-C), 170.4 (2-C)

### 2.3. Synthese von 4,6-Diethoxypyrimidin-2,5-diamin Dihydrochlorid (E2)

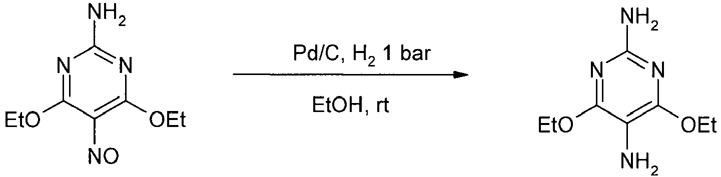

Zu einer Lösung von 14.1 g (0.066 mol) 2-Amino-4,6-diethoxy-5-nitrosopyrimidin in 400 ml Ethanol wurden 1.0 g (5%) Palladium auf Kohle gegeben und für 21 Stunden unter einem Wasserstoffdruck von 1 bar bei Raumtemperatur geschüttelt. Nach Beendigung der Reaktion wurde die Reaktionslösung auf 100 ml Salzsäure (10% in Wasser) gegossen, vom Katalysator abfiltriert und bis zur Trockene eingeengt. Das erhaltene gelbe Öl wurde mit Ethanol versetzt und erneut eingeengt. Der dann erhaltene Niederschlag wurde abgesaugt und mit wenig eiskaltem Ethanol nachgewaschen. Nach dem Trocknen erhielt man 4,6-Diethoxypyrimidin-2,5-diamin Dihydrochlorid (10.9 g, 61%) als hellgelben Feststoff.
¹H-NMR (300 MHz, d₆-DMSO): δ = 1.26 (t, 6 H, CH₃), 4.31 (q, 4 H, CH₂), 6.70 (brs, 4 H, NH₂). ¹³C-NMR (125 MHz, d₆-DMSO): δ = 14.8 (CH₃), 62.9 (-O-CH₂-), 87.0 (5-C), 161.1 (2-C), 163.8 (6-C, 4-C).

### 2. Färbebeispiele

### 2.1. Herstellung der Färbecremes

Es wurden die folgenden Färbecremes hergestellt:

| | |
|---|---|
| Hydrenol® D¹ | 8,5 Gew.-% |
| Lorol® tech.² | 2,0 Gew.-% |
| Texapon® NSO³ | 20,0 Gew.-% |
| Dehyton® K⁴ | 12,5 Gew.-% |
| Eumulgin® B2⁵ | 0,75 Gew.-% |
| Natriumsulfit | 1,0 Gew.-% |
| Ammoniumsulfat | 1,0 Gew.-% |
| Entwicklerkomponente | 3 mmol |
| Kupplerkomponente | 3 mmol |
| Wasser | ad 100 |

| | |
|---|---|
| ¹ C₁₆₋₁₈-Fettalkohol (INCI-Bezeichnung: Cetearyl alcohol) (Cognis) ² C₁₂₋₁₈-Fettalkohol (INCI-Bezeichnung: Coconut alcohol) (Cognis) ³ Laurylethersulfat, Natriumsalz (ca. 27,5% Aktivsubstanz; INCI-Bezeichnung: Sodium Laureth Sulfate) (Cognis) ⁴ N,N-Dimethyl-N-(C₈₋₁₈-kokosamidopropyl)ammoniumacetobetain (ca. 30% Aktivsubstanz; INCI-Bezeichnung: Aqua (Water), Cocamidopropyl Betaine) (Cognis) ⁵ Cetylstearylalkohol mit ca. 20 EO-Einheiten (INCI-Bezeichnung: Ceteareth-20) (Cognis) | |

Hydrenol D und Lorol techn wurden zusammen mit Texapon NSO, Dehyton K und Eumulgin B2 bei 80 °C aufgeschmolzen. Dann wurde die Schmelze mit dem in einem Teil des Wassers gelösten Natriumsulfit und Ammoniumsulfat emulgiert. Der erfindungsgemäße Entwickler wurde in einem weiteren Teil der angegebenen Wassermenge unter Erhitzen gelöst und unter Rühren hinzu gegeben. Der Kuppler wurde ebenfalls in einem Teil der angegebenen Wassermenge gelöst und unter Rühren hinzu gegeben. Dann wurde die Formulierung auf 100 % mit Wasser aufgefüllt und kalt gerührt.

Die auf diese Weise erhaltene Färbecreme wurde im Verhältnis 1:1 mit der folgenden Entwicklerdispersion mit einem Wasserstoffperoxidgehalt von 6% vermischt.

| | |
|---|---|
| Dipicolinsäure | 0,1 Gew.-% |
| Natriumpyrophosphat | 0,03 Gew.-% |
| Turpinal® SL⁶ | 1,50 Gew.-% |
| Texapon® N28⁷ | 2,00 Gew.-% |
| Acrysol® 22⁸ | 0,60 Gew.-% |
| Wasserstoffperoxid, 50 %ig | 6,00 Gew.-% |
| Natronlauge, 45%ig | 0,80 Gew.-% |
| Wasser | ad 100 Gew.-% |

| | |
|---|---|
| ⁶ 1-Hydroxyethan-1,1-diphosphonsäure (ca. 58 - 61% Aktivsubstanzgehalt; INCI-Bezeichnung: Etidronic Acid, Aqua (Water)) (Solutia) ⁷ Laurylethersulfat-Natrium-Salz (mind. 26,5 % Aktivsubstanzgehalt; INCI-Bezeichnung: Sodium Laureth Sulfate) (Cognis) ⁸ Acrylpolymer (ca. 29.5 - 30.5% Festkörper in Wasser; INCI-Bezeichnung: Acrylates/Steareth-20 Methacrylate Copolyme) | |

Für den Färbeprozess wurde jeweils auf eine Strähne zu 80 % ergrauten Haares (Kerling) die 4-fache Menge der anwendungsbereiten Mischung appliziert. Nach einer Einwirkzeit von 30 Minuten bei 32 °C wurden die Strähnen ausgespült und mit einem üblichen Haarwaschmittel ausgewaschen. Die Färbung der Strähnen wurde nach dem Trocknen visuell unter der Tageslichtlampe beurteilt. Die Färbeergebnisse sind in der nachfolgenden Tabelle zusammengefasst.

**Tabelle 1: Ausfärbungen mit 4,6-Dimethoxypyrimidin-2,5-diamin Dihydrochlorid (E1)**

| Beispiel | Kupplerkomponente | erhaltene Nuance / Farbintensität |
|---|---|---|
| 1 | Resorcin | graurot (+++) |
| 2 | 3-Amino-2-methylamino-6-methoxypyridin | sepia (+++) |
| 3 | 5-Amino-2-methylphenol | graumagenta (++) |
| 4 | 3-Amino-2-hydroxypyridin | rothaarig (+) |
| 5 | 1,3-Bis(2,4-diaminophenoxy)propan | dunkelmagenta (+++) |
| 6 | 1-Naphthol | mattviolett (++) |
| 7 | 2-Methylresorcin | braunrot (+++) |
| 8 | 1,5-Dihydroxynaphthalin | graurubin (++) |
| 9 | 1-Methyl-2,6-bis-(2-hydroxyethylamino)benzol | krapprot (+++) |

| | | |
|---|---|---|
| +++hohe Intensität ++ mittlere Intensität + niedrige Intensität | | |

**Tabelle 2: Ausfärbungen mit 4,6-Dimethoxypyrimidin-2,5-diamin Dihydrochlorid (E2)**

| Beispiel | Kupplerkomponente | erhaltene Nuance / Farbintensität |
|---|---|---|
| 1 | Resorcin | graurot (+++) |
| 2 | 3-Amino-2-methylamino-6-methoxypyridin | dunkelbraun (+++) |
| 3 | 5-Amino-2-methylphenol | rosenholz (++) |
| 4 | 3-Amino-2-hydroxypyridin | sahararot (++) |
| 5 | 1,3-Bis(2,4-diaminophenoxy)propan | dunkelpurpur (+++) |
| 6 | 1-Naphthol | mattviolett (++) |
| 7 | 2-Methylresorcin | graurot (+++) |
| 8 | 1,5-Dihydroxynaphthalin | purpurgrau (+++) |
| 9 | 1-Methyl-2,6-bis-(2-hydroxyethylamino)benzol | cerise (+++) |

| | | |
|---|---|---|
| +++ hohe Intensität ++ mittlere Intensität + niedrige Intensität | | |

## Patentansprüche

1. Mittel zur oxidativen Färbung keratinischer Fasern, enthaltend in einem kosmetischen Träger als Oxidationsfarbstoffvorprodukt vom Entwickler-Typ mindestens eine Verbindung der Formel (I), 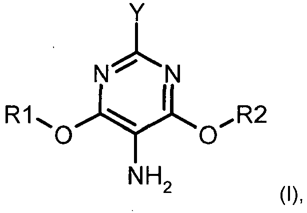 in der
R¹, R² unabhängig voneinander für eine C₁-C₆-Alkylgruppe, eine Hydroxy-C₁-C₆-alkylgruppe, eine C₁-C₆-Alkoxy-C₁-C₆-alkylgruppe, eine C₁-C₆-Alkanoylgruppe, eine C₁-C₆-Alkoxycarbonylgruppe, oder eine Arylgruppe stehen,
Y für eine Hydroxygruppe, eine Aminogruppe oder eine C₁-C₆-Alkylaminogruppe steht,
und/oder deren physiologisch verträgliches Salz.

2. Mittel nach Anspruch 1, **dadurch gekennzeichnet, dass** es mindestens eine Verbindung der Formel (I) enthält, in der R¹ und R² unabhängig voneinander für eine C₁-C₆-Alkylgruppe stehen.

3. Mittel nach einem der Ansprüche 1 bis 2, **dadurch gekennzeichnet, dass** es mindestens eine Verbindung der Formel (I) enthält, in der Y für eine Hydroxygruppe oder für eine Aminogruppe steht.

4. Mittel nach einem der Ansprüche 1 bis 3, **dadurch gekennzeichnet, dass** R¹ und R² beide für eine Methylgruppe stehen und Y für eine Aminogruppe steht.

5. Mittel nach einem der Ansprüche 1 bis 4, **dadurch gekennzeichnet, dass** es mindestens eine Verbindung der Formel (I) enthält, die ausgewählt ist aus 4,6-Dimethoxypyrimidin-2,5-diamin, 4-Methoxy-6-ethoxypyrimidin-2,5-diamin, 4,6-Diethoxypyrimidin-2,5-diamin, [(2,5-Diamino-6-methoxypyrimidin-4-yl)oxy]methanol, [(2,5-Diamino-6-ethoxypyrimidin-4-yl)oxy]methanol, [(2,5-Diamino-6-methoxypyrimidin-4-yl)oxy]ethanol, [(2,5-Diamino-6-ethoxypyrimidin-4-yl)oxy]ethanol, [(2,5-Diamino-6-methoxypyrimidin-4-yl)oxy]methoxy)methanol, [(2,5-Diamino-6-ethoxypyrimidin-4-yl)oxy]methoxy)methanol, 2-{2-[(2,5-Diamino-6-methoxypyrimidin-4-yl)oxy]ethoxy}ethan-1-ol, 2-{2-[(2,5-Diamino-6-ethoxypyrimidin-4-yl)oxy]ethoxy}ethan-1-ol und/oder deren physiologisch verträglichen Salzen.

6. Mittel nach einem der Ansprüche 1 bis 5, **dadurch gekennzeichnet, dass** es eine oder mehrere Verbindungen der Formel (I) in einer Gesamtmenge von 0,001 bis 5,0 Gew.-%, bevorzugt von 0,025 bis 2,5 Gew.-%, besonders bevorzugt von 0,05 bis 2,0 Gew.-% und insbesondere bevorzugt von 0,1 bis 1,5 Gew.-%, jeweils bezogen auf des Gesamtgewicht des anwendungsbereiten Mittels, enthält.

7. Mittel nach einem der Ansprüche 1 bis 6, **dadurch gekennzeichnet, dass** es zusätzlich mindestens ein Oxidationsfarbstoffvorprodukt vom Kupplertyp aus der Gruppe 3-Aminophenol, 5-Amino-2-methylphenol, 3-Amino-2-chlor-6-methylphenol, 2-(2,4-Diaminophenoxy)ethanol, 1,3-Bis(2,4-diaminophenoxy)propan, 1-Methoxy-2-amino-4-(2-hydroxyethylamino)benzol, 2,7-Dihydroxynaphthalin, 1,5- Dihydroxynaphthalin, 1-Naphthol, Resorcin, 2-Methylresorcin, 4-Chlorresorcin, 2-Amino-3-hydroxypyridin, 3-Amino-6-methoxy-2-methylaminopyridin, 2,6-Dihydroxy-3,4-dimethylpyridin, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-hydroxyethyl)amino]-2-methylphenyl}amino)ethanol, 2,6-Dihydroxy-3,4-dimethylpyridin 1-Methyl-2,6-bis(2-hydroxyethylamino)benzol und/oder einem physiologisch verträglichen Salz dieser Verbindungen enthält.

8. Mittel nach einem der Ansprüche 1 bis 7, **dadurch gekennzeichnet, dass** es zusätzlich
- ein oder mehrere Oxidationsfarbstoffvorprodukte vom Kupplertyp aus der Gruppe Resorcin, 2-Methylresorcin und/oder 4-Chlorresorcin enthält und
- ein oder mehrere Oxidationsfarbstoffvorprodukte vom Kupplertyp aus der Gruppe (2,4-Diaminophenoxy)ethanol, 1,3-Bis(2,4-diaminophenoxy)propan, 1-Methoxy-2-amino-4-(2-hydroxyethylamino)benzol enthält.

9. Mittel nach einem der Ansprüche 1 bis 8, **dadurch gekennzeichnet, dass** es zusätzlich
- ein oder mehrere Oxidationsfarbstoffvorprodukte vom Kupplertyp aus der Gruppe Resorcin, 2-Methylresorcin und/oder 4-Chlorresorcin enthält und
- ein oder mehrere Oxidationsfarbstoffvorprodukte vom Kupplertyp aus der Gruppe 2-Amino-3-hydroxypyridin, 3-Amino-6-methoxy-2-methylaminopyridin und/oder 2,6-Dihydroxy-3,4-dimethylpyridin enthält.

10. Mittel nach einem der Ansprüche 1 bis 9, **dadurch gekennzeichnet, dass** es zusätzlich
- ein oder mehrere Oxidationsfarbstoffvorprodukte vom Kupplertyp aus der Gruppe Resorcin, 2-Methylresorcin und/oder 4-Chlorresorcin enthält und
- ein oder mehrere Oxidationsfarbstoffvorprodukte vom Kupplertyp aus der Gruppe 1-Naphthol, 1,5-Dihydroxynaphthalin und/oder 1-Methyl-2,6-bis(2-hydroxyethylamino)benzol enthält.

11. Mittel nach einem der Ansprüche 1 bis 10, **dadurch gekennzeichnet, dass** es zusätzlich mindestens ein Oxidationsfarbstoffvorprodukt vom Entwicklertyp aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, 4-Aminophenol, 4-Amino-3-methylphenol, 4-Amino-2-aminomethylphenol, 4-Amino-2-(1,2-dihydroxyethyl)phenol und 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin benzol und/oder einem physiologisch verträglichen Salz dieser Verbindungen enthält.

12. Mittel nach einem der Ansprüche 1 bis 11, **dadurch gekennzeichnet, dass** es zusätzlich mindestens ein Oxidationsfarbstoffvorprodukt vom Entwicklertyp aus der Gruppe 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, 4,5-Diamino-1-(2-hydroxyethyl)pyrazol und/oder einem physiologisch verträglichen Salz dieser Verbindungen enthält.

13. Mehrkomponenten-Verpackungseinheit (Kit-of-parts) zum oxidativen Färben von keratinischen Fasern, umfassend zwei getrennt voneinander verpackte Mittel (M1) und (M2), wobei
- es sich bei dem Mittel (M1) um ein Mittel nach einem der Ansprüche 1 bis 12 handelt
- das Mittel (M2) in einem wässrigen kosmetischen Träger Wasserstoffperoxid enthält und
- die Mischung aus (M1) und (M2) Wasserstoffperoxid in einer Menge von 0,5 bis 6,0 Gew.-%, bevorzugt von 1,0 bis 5,0 Gew.-%, weiter bevorzugt von 1,5 bis 4,0 Gew.-% und besonders bevorzugt von 1,5 bis 3,0 Gew.-% - bezogen auf das Gesamtgewicht der Gemisches aus (M1) und (M2) - enthält.

14. Mehrkomponenten-Verpackungseinheit (Kit-of-parts) nach Anspruch 13, **dadurch gekennzeichnet, dass**
- die Mischung aus (M1) und (M2) Wasser in einer Menge von mind. 50 Gew.-%, bevorzugt von mind. 60 Gew.-%, weiter bevorzugt von mind. 70 Gew.-% und besonders bevorzugt von mind. 80 Gew.-% - bezogen auf das Gesamtgewicht der Gemisches aus (M1) und (M2) - enthält und
- die Mischung aus (M1) und (M2) einen pH-Wert von 7,0 bis 10,5, bevorzugt von 7,5 bis 9,5, weiter bevorzugt von 8,0 bis 9,0 besitzt

## Claims

1. An agent for oxidatively dyeing keratin fibers, containing in a cosmetic carrier at least one compound of formula (I) as a developer-type oxidation dye precursor, 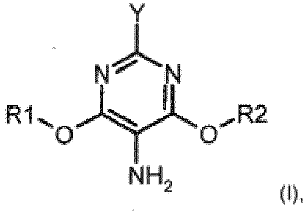 in which
R¹, R² represent, independently of one another, a C₁-C₆ alkyl group, a hydroxy-C₁-C₆ alkyl group, a C₁-C₆ alkoxy-C₁-C₆ alkyl group, a C₁-C₆ alkanoyl group, a C₁-C₆ alkoxycarbonyl group, or an aryl group,
Y represents a hydroxy group, an amino group, or a C₁-C₆ alkylamino group,
and/or the physiologically acceptable salt thereof.

2. The agent according to claim 1, **characterized in that** it contains at least one compound of formula (I), in which R¹ and R² represent, independently of one another, a C₁-C₆ alkyl group.

3. The agent according to one of claims 1 to 2, **characterized in that** it contains at least one compound of formula (I), in which Y represents a hydroxy group or an amino group.

4. The agent according to one of claims 1 to 3, **characterized in that** R¹ and R² both represent a methyl group and Y represents an amino group.

5. The agent according to one of claims 1 to 4, **characterized in that** it contains at least one compound of formula (I) selected from 4,6-dimethoxypyrimidine-2,5-diamine, 4-methoxy-6-ethoxypyrimidine-2,5-diamine, 4,6-diethoxypyrimidine-2,5-diamine, [(2,5-diamino-6-methoxypyrimidin-4-yl)oxy]methanol, [(2,5-diamino-6-ethoxypyrimidin-4-yl)oxy]methanol, [(2,5-diamino-6-methoxypyrimidin-4-yl)oxy]ethanol, [(2,5-diamino-6-ethoxypyrimidin-4-yl)oxy]ethanol, [(2,5-diamino-6-methoxypyrimidin-4-yl)oxy]methoxy)methanol, [(2,5-diamino-6-ethoxypyrimidin-4-yl)oxy]methoxy)methanol, 2-{2-[(2,5-diamino-6-methoxypyrimidin-4-yl)oxy]ethoxy}ethan-1-ol, 2-{2-[(2,5-diamino-6-ethoxypyrimidin-4-yl)oxy]ethoxy}ethan-1-ol and/or the physiologically acceptable salts thereof.

6. The agent according to one of claims 1 to 5, **characterized in that** it contains one or more compounds of formula (I) in a total amount of from 0.001 to 5.0 wt.%, preferably from 0.025 to 2.5 wt.%, particularly preferably from 0.05 to 2.0 wt.%, and more particularly preferably from 0.1 to 1.5 wt.%, in each case based on the total weight of the ready-to-apply agent.

7. The agent according to one of claims 1 to 6, **characterized in that** it additionally contains at least one coupler-type oxidation dye precursor from the group of 3-aminophenol, 5-amino-2-methylphenol, 3-amino-2-chloro-6-methylphenol, 2-(2,4-diaminophenoxy)ethanol, 1,3-bis(2,4-diaminophenoxy)propane, 1-methoxy-2-amino-4-(2-hydroxyethylamino)benzene, 2,7-dihydroxynaphthalene, 1,5-dihydroxynaphthalene, 1-naphthol, resorcinol, 2-methylresorcinol, 4-chlororesorcinol, 2-amino-3-hydroxypyridine, 3-amino-6-methoxy-2-methylaminopyridine, 2,6-dihydroxy-3,4-dimethylpyridine, 2-({3-[(2-hydroxyethyl)amino]-4-methoxy-5-methylphenyl}amino)ethanol, 2-({3-[(2-hydroxyethyl)amino]-2-methoxy-5-methylphenyl}amino)ethanol, 2-({3-[(2-hydroxyethyl)amino]-2-methylphenyl}amino)ethanol, 2,6-dihydroxy-3,4-dimethylpyridine, 1-methyl-2,6-bis(2-hydroxyethylamino)benzene, and/or a physiologically acceptable salt of these compounds.

8. The agent according to one of claims 1 to 7, **characterized in that** it additionally contains
- one or more coupler-type oxidation dye precursors from the group of resorcinol, 2-methylresorcinol and/or 4-chlororesorcinol, and
- one or more coupler-type oxidation dye precursors from the group of (2,4-diaminophenoxy)ethanol, 1,3-bis(2,4-diaminophenoxy)propane, 1-methoxy-2-amino-4-(2-hydroxyethylamino)benzene.

9. The agent according to one of claims 1 to 8, **characterized in that** it additionally contains
- one or more coupler-type oxidation dye precursors from the group of resorcinol, 2-methylresorcinol and/or 4-chlororesorcinol, and
- one or more coupler-type oxidation dye precursors from the group of 2-amino-3-hydroxypyridine, 3-amino-6-methoxy-2-methylaminopyridine and/or 2,6-dihydroxy-3,4-dimethylpyridine.

10. The agent according to one of claims 1 to 9, **characterized in that** it additionally contains
- one or more coupler-type oxidation dye precursors from the group of resorcinol, 2-methylresorcinol and/or 4-chlororesorcinol, and
- one or more coupler-type oxidation dye precursors from the group of 1-naphthol, 1,5-dihydroxynaphthalene and/or 1-methyl-2,6-bis(2-hydroxyethylamino)benzene.

11. The agent according to one of claims 1 to 10, **characterized in that** it additionally contains at least one developer-type oxidation dye precursor from the group of p-phenylenediamine, p-toluenediamine, 2-(2-hydroxyethyl)-p-phenylenediamine, 2-(1,2-dihydroxyethyl)-p-phenylenediamine, N,N-bis-(2-hydroxyethyl)-p-phenylenediamine, 2-methoxymethyl-p-phenylenediamine, N-(4-amino-3-methylphenyl)-N-[3-(1H-imidazol-1-yl)propyl]amine, N,N'-bis-(2-hydroxyethyl)-N,N'-bis-(4-aminophenyl)-1,3-diamino-propan-2-ol, bis-(2-hydroxy-5-aminophenyl)methane, 1,3-bis-(2,5-diaminophenoxy)-propan-2-ol, N,N'-bis-(4-aminophenyl)-1,4-diazacycloheptane, 1,10-bis-(2,5-diaminophenyl)-1,4,7,10-tetraoxadecane, 4-aminophenol, 4-amino-3-methylphenol, 4-amino-2-aminomethylphenol, 4-amino-2-(1,2-dihydroxyethyl)phenol and 4-amino-2-(diethylaminomethyl)-phenol, 4,5-diamino-1-(2-hydroxyethyl)pyrazole, 2,4,5,6-tetraaminopyrimidine, 4-hydroxy-2,5,6-triaminopyrimidine, 2-hydroxy-4,5,6-triaminopyrimidine benzene and/or a physiologically acceptable salt of these compounds.

12. The agent according to one of claims 1 to 11, **characterized in that** it additionally contains at least one developer-type oxidation dye precursor from the group of 2-(2-hydroxyethyl)-p-phenylenediamine, 2-methoxymethyl-p-phenylenediamine, N-(4-amino-3-methylphenyl)-N-[3-(1H-imidazol-1-yl)propyl]amine, bis-(2-hydroxy-5-aminophenyl)methane, 4,5-diamino-1-(2-hydroxyethyl)pyrazole, and/or a physiologically acceptable salt of these compounds.

13. A multicomponent packaging unit (kit of parts) for oxidatively dyeing keratin fibers, comprising two agents (M1) and (M2) that are separately packaged, wherein
- agent (M1) is an agent according to one of claims 1 to 12,
- agent (M2) contains hydrogen peroxide in an aqueous cosmetic carrier, and
- the mixture of (M1) and (M2) contains hydrogen peroxide in an amount of from 0.5 to 6.0 wt.%, preferably from 1.0 to 5.0 wt.%, more preferably from 1.5 to 4.0 wt.%, and particularly preferably from 1.5 to 3.0 wt.%, based on the total weight of the mixture of (M1) and (M2).

14. The multicomponent packaging unit (kit of parts) according to claim 13, **characterized in that**
- the mixture of (M1) and (M2) contains water in an amount of at least 50 wt.%, preferably at least 60 wt.%, more preferably at least 70 wt.%, and particularly preferably at least 80 wt.%, based on the total weight of the mixture of (M1) and (M2), and
- the mixture of (M1) and (M2) has a pH of 7.0 to 10.5, preferably 7.5 to 9.5, more preferably 8.0 to 9.0.

## Revendications

1. Agent de coloration d'oxydation de fibres kératiniques, contenant, dans un véhicule cosmétique, comme précurseur de colorant d'oxydation de type développeur, au moins un composé de formule (I), 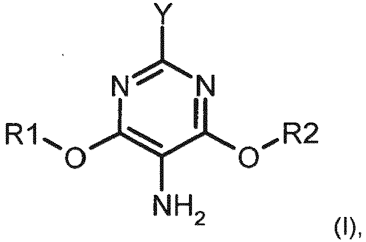 dans laquelle
R¹, R² représentent indépendamment l'un de l'autre un groupe alkyle en C₁-C₆, un groupe hydroxyalkyle en C₁-C₆, un groupe alcoxy en C₁-C₆alkyle en C₁-C₆, un groupe alcanoyle en C₁-C₆, un groupe alcoxycarbonyle en C₁-C₆ ou un groupe aryle,
Y représente un groupe hydroxy, un groupe amino ou un groupe alkylamino en C₁-C₆,
et/ou son sel physiologiquement compatible.

2. Agent selon la revendication 1, **caractérisé en ce qu'**il contient au moins un composé de formule (I), dans laquelle R¹ et R² représentent indépendamment l'un de l'autre un groupe alkyle en C₁-C₆.

3. Agent selon l'une des revendications 1 et 2, **caractérisé en ce qu'**il contient au moins un composé de formule (I), dans laquelle Y représente un groupe hydroxy ou un groupe amino.

4. Agent selon l'une des revendications 1 à 3, **caractérisé en ce que** R¹ et R² représentent tous les deux un groupe méthyle et Y, un groupe amino.

5. Agent selon l'une des revendications 1 à 4, **caractérisé en ce qu'**il contient au moins un composé de formule (I) sélectionné parmi la 4,6-diméthoxypyrimidine-2,5-diamine, la 4-méthoxy-6-éthoxypyrimidine-2,5-diamine, la 4,6-diéthoxypyrimidine-2,5-diamine, le [(2,5-diamino-6-méthoxypyrimidin-4-yl)oxy]méthanol, le [(2,5-diamino-6-éthoxypyrimidin-4-yl)oxy]méthanol, le [(2,5-diamino-6-méthoxypyrimidin-4-yl)oxy]éthanol, le [(2,5-diamino-6-éthoxypyrimidin-4-yl)oxy]éthanol, le [(2,5-diamino-6-méthoxypyrimidin-4-yl)oxy]méthoxy)méthanol, le [(2,5-diamino-6-éthoxypyrimidin-4-yl)oxy]méthoxy)méthanol, le 2-{2-[(2,5-diamino-6-méthoxypyrimidin-4-yl)oxy]éthoxy}éthan-1-ol, le 2-{2-[(2,5-diamino-6-éthoxypyrimidin-4-yl)oxy]éthoxy}éthan-1-ol, et/ou leurs sels physiologiquement compatibles.

6. Agent selon l'une des revendications 1 à 5, **caractérisé en ce qu'**il contient un ou plusieurs composés de formule (I) en une quantité totale de 0,001 à 5,0 % en poids, de préférence de 0,025 à 2,5 % en poids, de manière particulièrement préférée de 0,05 à 2,0 % en poids et de manière tout particulièrement préférée de 0,1 à 1,5 % en poids, par rapport au poids total de l'agent prêt à l'emploi respectivement.

7. Agent selon l'une des revendications 1 à 6, **caractérisé en ce qu'**il contient en plus au moins un précurseur de colorant d'oxydation de type coupleur issu de l'ensemble comprenant le 3-aminophénol, le 5-amino-2-méthylphénol, le 3-amino-2-chloro-6-méthylphénol, le 2-(2,4-diaminophénoxy)éthanol, le 1,3-bis(2,4-diaminophénoxy)propane, le 1-méthoxy-2-amino-4-(2-hydroxyéthylamino)benzol, le 2,7-dihydroxynaphtalène, le 1,5-dihydroxynaphtalène, le napht-1-ol, le résorcinol, le 2-méthylrésorcinol, le 4-chlororésorcinol, la 2-amino-3-hydroxypyridine, la 3-amino-6-méthoxy-2-méthylaminopyridine, la 2,6-dihydroxy-3,4-diméthylpyridine, le 2-({3-[(2-hydroxyéthyl)amino]-4-méthoxy-5-méthylphényl}amino)éthanol, le 2-({3-[(2-hydroxyéthyl)amino]-2-méthoxy-5-méthylphényl}amino)éthanol, le 2-({3-[(2-hydroxyéthyl)amino]-2-méthylphényl}amino)éthanol, la 2,6-dihydroxy-3,4-diméthylpyridine, le 1-méthyl-2,6-bis(2-hydroxyéthylamino)benzène et/ou un sel physiologiquement compatible de ces composés.

8. Agent selon l'une des revendications 1 à 7, **caractérisé en ce qu'**il contient en plus
- un ou plusieurs précurseurs de colorant d'oxydation de type coupleur issus de l'ensemble comprenant le résorcinol, le 2-méthylrésorcinol et/ou le 4-chlororésorcinol, et
- un ou plusieurs précurseurs de colorant d'oxydation de type coupleur issu de l'ensemble comprenant le (2,4-diaminophénoxy)éthanol, le 1,3-bis(2,4-diaminophénoxy)propane, le 1-méthoxy-2-amino-4-(2-hydroxyéthylamino)benzène.

9. Agent selon l'une des revendications 1 à 8, **caractérisé en ce qu'**il contient en plus
- un ou plusieurs précurseurs de colorant d'oxydation de type coupleur issus de l'ensemble comprenant le résorcinol, le 2-méthylrésorcinol et/ou le 4-chlororésorcinol, et
- un ou plusieurs précurseurs de colorant d'oxydation de type coupleur issu de l'ensemble comprenant la 2-amino-3-hydroxypyridine, la 3-amino-6-méthoxy-2-méthylaminopyridine et/ou la 2,6-dihydroxy-3,4-diméthylpyridine.

10. Agent selon l'une des revendications 1 à 9, **caractérisé en ce qu'**il contient, en plus,
- un ou plusieurs précurseurs de colorant d'oxydation de type coupleur issus de l'ensemble comprenant le résorcinol, le 2-méthylrésorcinol et/ou le 4-chlororésorcinol, et
- un ou plusieurs précurseurs de colorant d'oxydation de type coupleur issu de l'ensemble comprenant le napht-1-ol, le 1,5-dihydroxynaphtalène et/ou le 1-méthyl-2,6-bis(2-hydroxyéthylamino)benzène.

11. Agent selon l'une des revendications 1 à 10, **caractérisé en ce qu'**il contient en plus au moins un précurseur de colorant d'oxydation de type développeur issu de l'ensemble comprenant la p-phénylènediamine, la p-toluylènediamine, la 2-(2-hydroxyéthyl)-p-phénylènediamine, la 2-(1,2-dihydroxyéthyl)-p-phénylènediamine, la N,N-bis-(2-hydroxyéthyl)-p-phénylènediamine, la 2-méthoxyméthyl-p-phénylènediamine, la N-(4-amino-3-méthylphényl)-N-[3-(1H-imidazol-1-yl)propyl]amine, le N,N'-bis-(2-hydroxyéthyl)-N,N'-bis-(4-aminophényl)-1,3-diamino-propan-2-ol, le bis-(2-hydroxy-5-aminophényl)méthane, le 1,3-bis-(2,5-diaminophénoxy)-propan-2-ol, le N,N'-bis-(4-aminophényl)-1,4-diazacycloheptane, le 1,10-bis-(2,5-diaminophényl)-1,4,7,10-tétraoxadécane, le 4-aminophénol, le 4-amino-3-méthylphénol, le 4-amino-2-aminométhylphénol, le 4-amino-2-(1,2-dihydroxyéthyl)phénol et le 4-amino-2-(diéthylaminométhyl)-phénol, le 4,5-diamino-1-(2-hydroxyéthyl)pyrazol, la 2,4,5,6-tétraaminopyrimidine, la 4-hydroxy-2,5,6-triaminopyrimidine, la 2-hydroxy-4,5,6-triaminopyrimidine, le benzène et/ou un sel physiologiquement compatible de ces composés.

12. Agent selon l'une des revendications 1 à 11, **caractérisé en ce qu'**il contient en plus au moins un précurseur de colorant d'oxydation de type développeur issu de l'ensemble comprenant la 2-(2-hydroxyéthyl)-p-phénylènediamine, la 2-méthoxyméthyl-p-phénylènediamine, la N-(4-amino-3-méthylphényl)-N-[3-(1H-imidazol-1-yl)propyl]amine, le bis-(2-hydroxy-5-aminophényl)méthane, le 4,5-diamino-1-(2-hydroxyéthyl)pyrazol, et/ou un sel physiologiquement compatible de ces composés.

13. Unité d'emballage à composants multiples (Kit-of-parts) pour la coloration d'oxydation de fibres kératiniques, comprenant deux agents (M1) et (M2) conditionnés séparément l'un de l'autre,
- l'agent (M1) correspondant à un agent selon l'une des revendications 1 à 12,
- l'agent (M2) contenant du peroxyde d'hydrogène dans un véhicule cosmétique aqueux, et
- le mélange de (M1) et (M2) contenant du peroxyde d'hydrogène en une quantité de 0,5 à 6,0 % en poids, de préférence de 1,0 à 5,0 % en poids, de manière davantage préférée de 1,5 à 4,0 % en poids, et de manière particulièrement préférée de 1,5 à 3,0 % en poids, par rapport au poids total du mélange de (M1) et (M2).

14. Unité d'emballage à composants multiples (Kit-of-parts) selon la revendication 13, **caractérisée en ce que**
- le mélange de (M1) et (M2) contient de l'eau en une quantité d'au moins 50% en poids, de préférence d'au moins 60% en poids, de manière davantage préférée d'au moins 70% en poids, et de manière particulièrement préférée d'au moins 80 % en poids, par rapport au poids total du mélange de (M1) et (M2), et
- le mélange de (M1) et (M2) possède un pH allant de 7,0 à 10,5, de préférence de 7,5 à 9,5, de manière davantage préférée de 8,0 à 9,0.
